# EUROPEAN PATENT APPLICATION

(11) **EP 2 033 649 A1**
(43) Date of publication of application: **11.03.2009**
(21) Application number: 08380005.2
(22) Date of filing: 10.01.2008
(51) Int. Cl.: A61K 31/535, A61K 31/498, A61K 31/382, A61P 27/06

(54) **Pharmaceutical composition for treatment of ocular hypertension**

(30) Priority: 17.08.2007 MX PA07010025
(71) Applicant: Jimenez Bayardo, Arturo, Guadalajara, Jalisco 44100 (MX)
(72) Inventor: Tornero Montaño, Jose Ruben, Guadalajara Jalisco 44100 (MX); Baiza Duran, Leopoldo Martin, Guadalajara Jalisco 44100 (MX); Quintana Hau, Juan de Dios, Guadalajara Jalisco 44100 (MX)
(74) Representative: Temino Ceniceros, Ignacio

(57) **Abstract**

The present invention is related to the pharmaceutical industry in general and in particular with the pharmaceutical industry of production of ophthalmic compositions. More specifically, the present invention relates to the pharmaceutical industry of production of ophthalmologic compositions for the treating of ocular hypertension. The advantage of the present invention against the present state of the technique is the fulfilling of a synergic effect of its components for the decreasing of ocular hypertension with no antagonistic effect between its components. The present invention consists of a pharmaceutical composition for the treatment of ocular hypertension characterized by comprising a pharmacologically effective amount of dorzolamide, with a pharmacologically effective amount of timolol and a pharmacologically effective amount of brimonidine.

## Description

### FIELD OF THE INVENTION

The present invention is related to the pharmaceutical industry in general and in particular to the pharmaceutical industry of ophthalmic composition production. More specifically, the present invention relates to the pharmaceutical industry of production of ophthalmic drugs for the treatment of ocular hypertension.

### BACKGROUND OF THE INVENTION

There are many therapeutical options to achieve the ideal intraocular pressure for when the therapy with beta-blockers fails. Two of the most frequent options are the fixed combination with dorzolamide/timolol.

If the starting therapy is successful but further decrease of intraocular pressure is required, a topical drug is added to the beta-blocker as an alpha2-adregenic antagonist, a topic inhibitor of the carbonic anhydrase or an analog of the prostaglandins, individually according to the patient's features and his clinical condition. Afterwards, if the first combination of drugs has an additive effect but does not completely fulfill the clinical objectives, another drug may be added. This process can be carried on until a minimum increase of benefits and a maximum increase in cost and inconvenience for the patient are obtained. ((10) Marchetti A, Magar R, An P. Clinical and Economic Impact of new trends in glaucoma treatment Medscape General Medicine July, 2001)

Dorzolamide is a topical carbonic anhydrase inhibitor which reduces intraocular pressure by reducing the production of the aqueous humour. It is frequently prescribed as joint therapy to timolol to reach an additional decrease in intraocular pressure. In controlled clinical studies, dorzolamide provides a reduction in additional intraocular pressure, being this added effect clinically significant from 13 to 21% in the reduction of intraocular pressure when added to beta-blockers. ((11) Pfeiffer N. Dorzolamide: Development and Clinical Application of a topical carbonic anhydrase inhibitor. Survey Ophthalmology 1997; 42(2):137-151)

The added effect in diminishing intraocular pressure of two or more drugs must be expected when these drugs are used together as medicines that act by different means. The carbonic anhydrase inhibitors are additives to beta-adrenergic blocking agents despite their mutual inhibition of aqueous humour production, since the means of action of the beta blocker do not reduce the carbonic anhydrase's activity. ((12) Brubaker R, Ingram C, Schoff E, et al. Comparison of the efficacy of betaxolol-brinzolamide and timolol-dorzolamide as suppressors of aqueous humor flow in human subjects. Ophthalmology 2000; 107:283-287)

Brimonidine tartrate is a highly selective alpha-2 adrenergic antagonist ocular antihypertensive drug. Despite the fact that brimonidine is structurally related to clonidine, brimonidine has a higher selectivity to alpha-2-andrenergic receptors. Some studies suggest that brimonidine may be used as a joint treatment to topical beta-blocker therapy, producing an additive effect of intraocular pressure reduction from 5 and 3.7mmhg. ((13) A Useful New Topical treatment for glaucoma and ocular hypertension. Drug Ther Perspect 13(1):1-4 1999)

Dorzolamide at 2% and brimonidine at 0.2% have well documented efficiency and tolerance profiles. When used as joint therapy with topical beta-blockers therapy in adults with ocular hypertension, brimonidine at 0.2% was found by Simmons to be more effective in reducing ocular hypertension than dorzolamide at 2%. Alternatively, Cantor and his collaborators found no statistically significant differences in the effectiveness of brimonidine and dorzolamide jointly used in patients with ocular hypertension using topical beta-blockers. ((14) Whitson J, Henry C, Hughes B. Comparison of the safety and efficacy of dorzolamide 2% and brimonidine 0.2% in patients with glaucoma or ocular hypertension. J Glaucoma 2004; 13:168-173)

Ermis and his collaborators studied the ocular antihypertensive effect of dorzolamide at 2% in patients with intraocular pressure of at least 22 mm Hg treated with topical brimonidine, thus proving the additional reducing effect of dorzolamide in said patients, reducing up to 29% in 30 days.

Brimonidine and dorzolamide used individually as single therapy have a similar ocular antihypertensive effect. It has been reported that dorzolamide reduces intraocular pressure by 20% when added to timolol, thus indicating a good additive effect of dorzolamide and timolol. Brimonidine reduces intraocular pressure by reducing the production of aqueous humour and increasing the flow through the uveoscleral routes. Brimonidine and dorzolamide can be an effective mixture. ((15) Ermis S, Ozturk F, Ubeyt Umit. The short term PIO-lowering effect of brimonidine 0.2% and dorzolamide 0.2% combination in primary open-angle glaucoma. Acta Ophthalmol Scand. 2002:80:632-634)

The success of glaucoma treatment is based not only on the efficiency, but also on the side effects and the patients fulfilling of the treatment. The prevalence of not fulfilling, or failing to take the medicine as prescribed, is high in glaucoma patients, ranging from 27% to 56%, depending on the population and the method of measuring data. With new ophthalmic products being available, the maximum level of medical therapy can become more complex due to the fact that new products have different time of action and prescription schemes. Its joint use with other topical agents can take from 30 to 60 minutes two times a day for proper use. This type of treatments can have serious economic implications for the patients. The more complex the treatment, the less it is fulfilled by the patient. ((10 Marchetti op cit.)

Added to this, numerous studies have shown that chronic use of antihypertensive drugs may cause significant changes on the eye's surface due to the fact that prolonged use of topical drugs containing preservatives may induce changes on the eye's surface and damage conjunctival and corneal epithelial cells, and also cause abnormal infiltration of inflammatory cells and/or fibroblast markers. ((16) Noecker R, Herrygers L, Anwaruddin R. Corneal and conjunctival changes caused by commonly used glaucoma medications. Corna 2004;23:490-496; 17) Baudouin C, Pisella PJ, Fillacier K, et al. Ocular surface inflammatory changes induced by topical antiglaucoma drugs. Ophthalmology.1999; 106:556-563)

Historically, additional agents were administered with the beta-blockers in a concomitant fashion. Never the less, an actual tendency in the development of glaucoma drugs has been the fixed combination of two agents.

Januleviciene and collaborators (Januleviciene I, Harris A, Kagemann L, et al. A comparison of the effects of dorzolamide/timolol fixed combination versus latanoprost on intraocular pressure and pulsatile ocular blood flow in primary open-angle glaucoma patients. Act Ophthalmol Scand. 2004;82:730-737) took on a study to analyze the effects of the dorzolamide/timolol fixed combination versus latanoprost on intraocular pressure and pulsatile ocular blood flow in 30 primary open-angle glaucoma patients (20). The dorzolamide/timolol fixed combination and latanoprost showed a statistically significant reduction of intraocular pressure, 4.6 mmHg and 3.75 mmHg respectively, and presented an increase in pulsating ocular blood flow of 2.048 MI/second and 2.147MI/second respectively.

Timolol is a non-selective B1 and B2 beta-adrenergic blocking agent which reduces high and regular intraocular pressure. The start of the drug's action with one drop of it can be detected within a single hour of topical ocular instillation, with a peak effect observed within 2 to 4 hours. A significant reduction of intraocular pressure can be maintained for periods of up to 24 hours with a single dose. The drug can be systemically absorbed and cause a decrease in heart rate, cardiac arrhythmia and bronchial spasm. Timolol has little or no effect over pupil size or accommodation. (1,7)

It has not been possible to explain the degree of systemic absorption that takes place after topical application of timolol. After the topical ocular administration of one drop of timolol at 0.5%, the start of the drugs action can be detected after an hour of topical ocular instillation. The peak effect of timolol happens after 2 hours and said effect lasts over 24 hours. The efficiency of these drugs can be diminished with time (tachyphylaxis), though the ocular antihypertensive effect can been maintained successfully for up to three years. The beta-adrenergic antagonists do not reduce intraocular pressure during sleep.

When administered topically, dorzolamide reaches systemic circulation and joins the erythrocytes quickly. The human erythrocytes contain carbonic anhydrase I and II isoenzymes. Liquid chromatography has identified a metabolite of dorzolamide, deethylized n-dorzolamide. Dorzolamide may a greater affinity for the isoenzyme II of carbonic anhydrase while the n-deethylized metabolite has a greater affinity for carbonic anhydrase I. Dorzolamide joins the plasma proteins moderately (aprox. 33%). After 4 weeks of bilateral treatment, the carbonic anhydrase isoenzyme's activity decreases by 21 % of its basic activity.

Fluor photometric studies in animals and humans suggest that the brimonidine tartrate solution reduces intraocular pressure by decreasing the production of aqueous humour and increasing the uveoscleral flow. The peak ocular antihypertensive effects take place 2-3 hours after the topical administration of the ophthalmic brimonidine tartrate solution.

The use of joint therapies presents serious deficiencies when used one application after the other. Combinations of carbonic anhydrase inhibitors and beta-adrenergic blocking agents have been suggested for the treatment of some ophthalmic pathologies.

It has not been determinately proved that all carbonic anhydrase inhibitors and all beta-adrenergic blocking agents are efficient for the treatment of ocular hypertension.

On the other hand, it has not been proved either if any antagonistic effect is presented in the action of the combination of these types of agents.

Reactions between said components while making the composition have not been studies either.

### OBJECTIVES OF THE INVENTION

One of the objectives of this invention is to achieve a composition of its components with synergic effect in order to decrease ocular hypertension.

Another objective is to determine the quantitative composition of these drugs.

Yet another objective is to determine whether any chemical reactions that produce modifications in the active molecules take place in the combinations that achieve the first objective.

One more objective of the present invention to prove there is no antagonistic effect between the components.

And all those objectives which will become apparent with the present description and the included annexes.

### BRIEF DESCRIPTION OF THE INVENTION

In brief, the present invention consists of a novelty qualitative composition for the treatment of ocular hypertension consisting of the combination of dorzolamide, timolol and brimonidine.

In one of its methods, the present invention consists of a composition of timolol at 0.5%, dorzolamide at 2% and brimonidine at 0.2%.

The choosing of these components took place by the determining of which products are more efficient and effective in their sequential use.

Dorzolamide, timolol and brimonidine have been formulated as a combined product which will provide a more convenient regimen for the patients who require multiple medications. The decrease in the amount of products and installments required daily may increase the degree of fulfillment and reduce the dilution effect of two different drops applied immediately one after another, consequently increasing the control over intraocular pressure, and also reducing the risk brought upon by chronic use of preservative-containing multidose ophthalmic solutions.

Subsequent to this determination, the efficiency of these combinations was assessed to determine the non-competition between them in their effect on ocular pressure.

Also determined was the non-reaction of the molecules amongst each other while being combined.

As a collateral determination the evolution of red-eye severity index and index of improvement in burning sensation were analyzed to assess said composition.

For a better understanding of the invention, the detailed description of the present invention shall now be made, showing the results of various tests made with the selected composition.

### BRIEF DESCRIPTION OF THE DRAWINGS

Figure 1 shows a graph of the evolution of eye pressure on the Y axis against time on the X axis for the treatments with the mixture timolol at 0.5%, dorzolamide at 2% and brimonidine at 0.2%.
Figure 2 shows a graph of the evolution of red-eye severity index on the Y axis against time on the X axis.
Figure 3 shows a graph of improvement in burning sensation on the Y axis against time in the X axis.
Figure 4 shows the chromatogram where the peaks of brimonidine and dorzolamide from an initial standard were imprinted.
Figure 5 shows the chromatogram of dorzolamide and brimonidine from a freshly prepared lot of dorzolamide, brimonidine and timolol.
Figure 6 shows the chromatogram of running the standards at the time the running of the same lot was made, the later was used to determine the chromatogram in figure 5, six months later.
Figure 7 shows the chromatogram of the same lot which wielded as a result the graph with the peaks in figure 5, but six months after.
Figure 8 shows the chromatogram with the timolol peak at a standard at the moment of the initial valuation of timolol in the lot of the previous chromatograms.
Figure 9 shows the chromatogram with the timolol peak in a recently prepared lot of dorzolamide, brimonidine and timolol, which is the same lot of the different chromatograms where lot is mentioned.
Figure 10 is the chromatogram of the same lot on which the run was made for the chromatogram in figure 9, but six months later.

For a better understanding of the invention, a detailed description of the invention will now be made showing the best way to accomplish the invention and fulfilling the characteristics of habilitation of a third party with average knowledge in the subject.

### DETAILED DESCRIPTION OF THE INVENTION

A comparative multicentre study of the safety and efficiency of a sterile ophthalmic solution based on timolol at 0.5, dorzolamide at 2% brimonidine tartrate at 0.2%.

The purpose of the investigation was to assess the safety, tolerance and efficiency of an ophthalmic solution of timolol at 0.5, dorzolamide at 2% and brimonidine tartrate at 0.2% in fixed combination (Krytantek Ofteno®) produced by Laboratorios Sophia SA. de C.V. and compare them with those of a topical solution of dorzolamide and timolol (Cosopt®) in patients diagnosed with open angle primary glaucoma and/or ocular hypertension with or without pseudoexfoliation.

### MATERIAL AND METHODS

A multicentre study was made, prospective, random and double-blind, where patients diagnosed with open angle primary glaucoma and/or ocular hypertension were selected and divided into two treatment groups which were assessed during a period of three months. During this time, one group randomly and blindly received one drop of Krytantek Ofteno® (timolol at 0.5%, dorzolamide at 2% and brimonidine at 0.2%, Laboratorios Sophia) every 12 hours, while the other group was treated in the same conditions with Cosopt® (timolol at 0.5% and dorzolamide at 2%, Laboratorios MSD). The efficiency and safety parameters assessed included visual acuteness (LogMar), intraocular pressure with a Goldmann tonometer, assessment of visual fields with Humphrey perimetry (strategy 24-2 white/white), dyeing with rose Bengal dye and fluorescein, conjunctival hyperemia, assessment of the corneal surface, pain, photophobia and foreign body sensation. The study's results were submitted to an inferential statistic analysis using Student's t-tests for independent samples, Wilcoxon's test, U de Mann-Whitney's U test and the Ji-Square Test.

### RESULTS

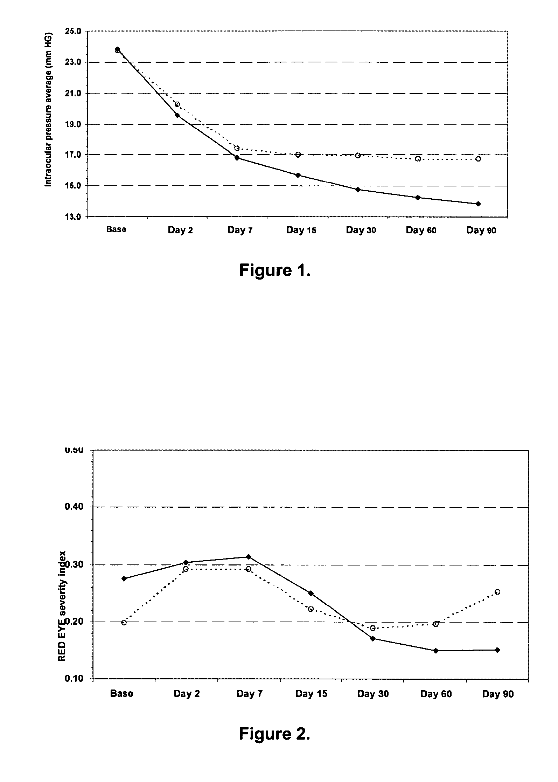

With 12 different research centres working together, a total of 104 patients were included (25.5% men and 74.5% women), with an average age of 58. Using Mann-Whitney's test, an important decrease in intraocular pressure was observed with both medications, marking significant differences when compared with their own average base values (intra-group), while the behavior of these values compared to the group which received Krytantek Ofteno® showed statistically significant differences (p<0.05) compared to Cosopt® (Figure 1), in favor of Krytantek Ofteno®.

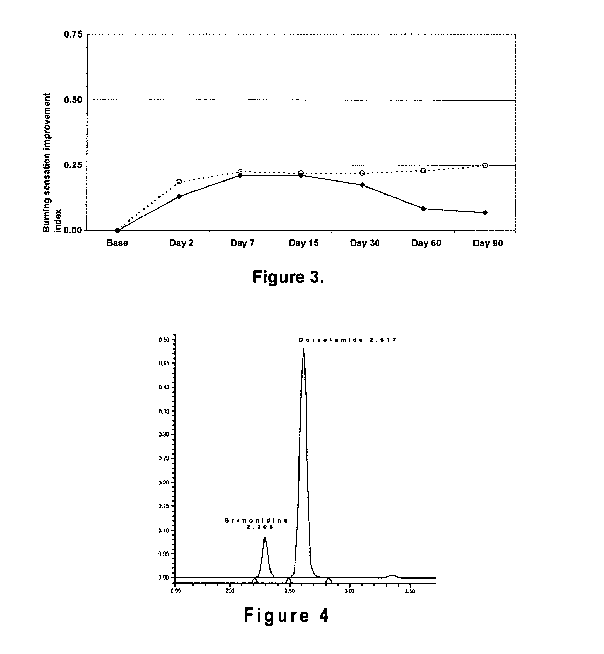

Regarding visual acuteness, dyeing with rose Bengal dye and fluorescein, evolution of the visual fields, assessment of the corneal surface, photophobia and foreign body sensation, no statistically significant differences were presented between both groups throughout the treatment. Nevertheless, regarding burning sensation and conjunctival hyperemia, the treatment group of Krytantek Ofteno showed lower values when compared with Cosopt (Ji-Square p<0.05), by day 90 of the treatment (Figures 2 and 3).

### CONCLUSIONS:

According to the results obtained, we can conclude that Krytantek Ofteno^{®} is more effective than Cosopt^{®} in reducing intraocular pressure in open angle primary Glaucoma and/or ocular hypertension patients, showing also a statistically significant tendency of higher tolerance in patients using Krytantek Ofteno^{®}.

With regards to the stability of the different components of the composition subject of the present description, different runs were made in the chromatograph to measure distinct changes in the molecules.

For the runnings, a standard is always required to verify the coincidence of the peaks. In Figure 4 a chromatogram is shown where the peaks of brimonidine and dorzolamide were imprinted from an initial standard. This was a secondary standard from a USP standard.

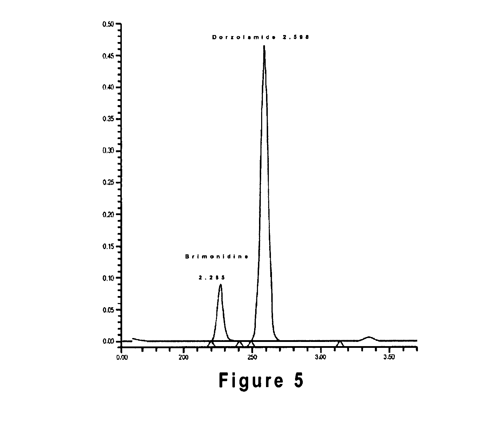

Figure 5 is from the chromatogram of dorzolamide and brimonidine from a freshly prepared lot of dorzolamide, brimonidine and timolol. This is the lot to which 6 months later the same determination was made.

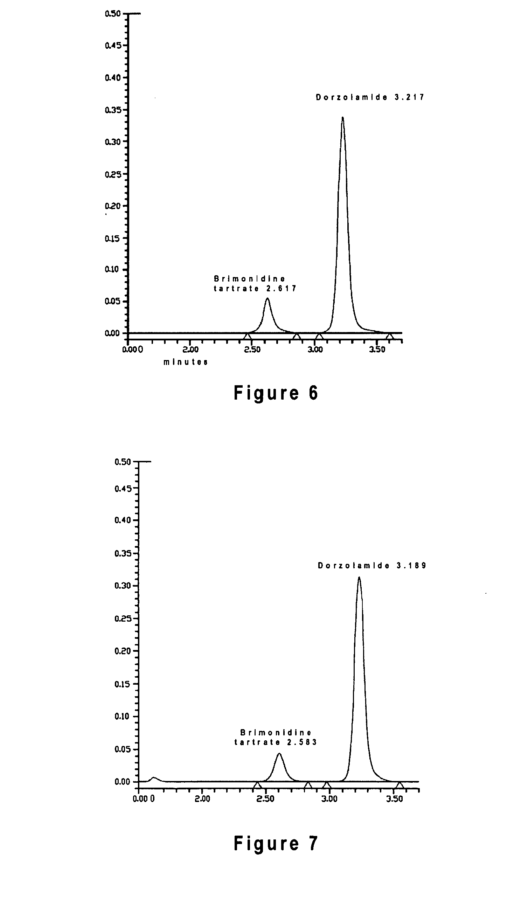

Figure 6 shows the chromatogram of running the standards at the time the running of the same lot was made, the later was used to determine the chromatogram in figure 5, six months later. This standard was prepared at the moment of the runnings to minimize errors.

Figure 7 shows the chromatogram of the same lot which wielded as a result the graph with the peaks in figure 5, but six months after. The lot was kept at extreme conditions to correctly assess the stability of the different molecules.

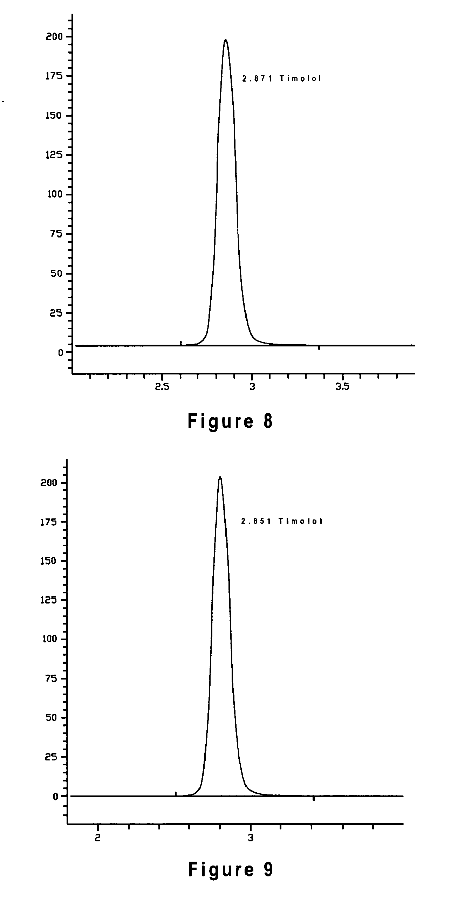

Figure 8 shows the chromatogram with the timolol peak at a standard at the moment of the initial valuation of timolol in the lot of the previous chromatograms.

Figure 9 shows the chromatogram with the timolol peak in a recently prepared lot of dorzolamide, brimonidine and timolol, which is the same lot of the different chromatograms where lot is mentioned. The separation of this chromatogram from the rest was done for a better clarity of the diagram.

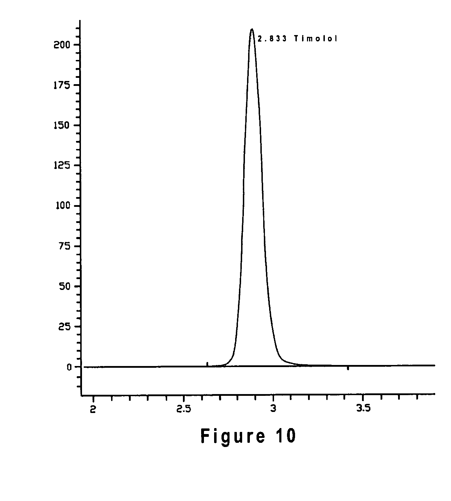

Figure 10 is the chromatogram of the same lot on which the run was made for the chromatogram in figure 9, but six months later.

The invention has been sufficiently described so that a person with average understanding of the subject can reproduce it and obtain the results mentioned in the present invention. Nevertheless, any person skillful in the field of the technique competing the present invention may be able to make undescribed modifications in the present application. However, if for the application of said modifications in a determined composition the material claimed in the following claims is needed, said processes and solutions must be comprised within the reach of the present invention.

## Claims

1. A pharmaceutical composition for the treatment of ocular hypertension **characterized by** comprising a pharmacologically effective amount of dorzolamide, with a pharmacologically effective amount of timolol and a pharmacologically effective amount of brimonidine.

2. A pharmaceutical composition for the treatment of ocular hypertension as claimed in the previous claim, **characterized by** the percentage of dorzolamide being 2% weight/weight with regards to the final composition.

3. A pharmaceutical composition for the treatment of ocular hypertension as claimed in claims 1 or 2, **characterized by** the percentage of timolol being 0.5% weight/weight with regards to the final composition.

4. A pharmaceutical composition for the treatment of ocular hypertension as claimed in any of claims 1 to 3, **characterized by** the percentage of brimonidine being 0.2% weight/weight with regards to the final composition.

5. A pharmaceutical composition for the treatment of ocular hypertension as claimed in claim 1, **characterized by** the percentage of dorzolamide being 2% weight/weight with regards to the final composition; the percentage of timolol being 0.5% weight/weight with regards to the final composition and the percentage of brimonidine being 0.2% weight/weight with regards to the final composition.
